# EUROPÄISCHE PATENTSCHRIFT

(11) **EP 1 787 605 B1**
(45) Veröffentlichungstag und Bekanntmachung des Hinweises auf die Patenterteilung: **13.10.2010**
(21) Anmeldenummer: 06023786.4
(22) Anmeldetag: 16.11.2006
(51) Int. Cl.: B65D 83/00, B65D 47/20

(54) **Spender und Dosierbaugruppe für die Mediumdosierung**
Dispenser and dosing set for the medium dosage
Distributeur et ensemble de dosage pour la dosage d'une substance

(30) Priorität: 21.11.2005 DE 10556488
(43) Veröffentlichungstag der Anmeldung: 23.05.2007
(73) Patentinhaber: Ing. Erich Pfeiffer GmbH, 78315 Radolfzell (DE)
(72) Erfinder: Fuchs, Karl-Heinz, 78315 Radolfzell (DE)
(74) Vertreter: Patentanwälte Ruff, Wilhelm, Beier, Dauster & Partner

(56) Entgegenhaltungen:
- EP-A- 0 388 741
- EP-A- 1 136 387
- DE-A1- 1 482 675
- DE-A1- 2 822 115
- FR-A- 2 206 741
- FR-A- 2 709 734
- GB-A- 726 756

## Beschreibung

Die Erfindung betrifft eine Dosierbaugruppe für einen Spender.

Aus dem Stand der Technik sind Spender bekannt, die für die Dosierung von flüssigen oder pulverförmigen Medien, insbesondere von pharmazeutisch wirksamen Substanzen vorgesehen sind und die eine manuelle Dosierung des in einem Mediumspeicher aufgenommenen Mediums ermöglichen. Derartige Spender ermöglichen eine Mediumdosierung in Form von Tropfen, Tröpfchennebel oder Pulvernebel und werden insbesondere dazu eingesetzt, um Medien in natürliche Körperöffnungen wie Augen, Ohren oder Nase einzubringen. Aus dem Stand der Technik ist es insbesondere bekannt, einen derartigen Spender als Pipette für Nasentropfen, als Quetschflasche für Augen- oder Ohrentropfen oder als kompressible Kunststoffampulle für eine Einmaldosierung von Medien auszuführen. Speziell im Bereich der Verabreichung von Augentropfen sind die aus dem Stand der Technik bekannten Spender ungünstig in der Handhabung, da sie erhebliche Bedienkräfte erfordern und in Anbetracht einer meist schlanken, im Wesentlichen zylindrischen Geometrie nur unzureichend für eine zielgenaue Dosierung und Applikation des Mediums geeignet sind.

Die Aufgabe der Erfindung besteht darin, eine Dosierbaugruppe zu schaffen, die eine präzisere Dosierung sowie eine einfache Handhabung, insbesondere zur Verabreichung von Augentropfen, ermöglichen.

Die der Erfindung zugrunde liegende Aufgabe wird durch eine Dosierbaugruppe mit einem Ventilgehäuse gelöst, das in einem Wandbereich von einer Dosieröffnung durchdrungen ist, die von einem im Ventilgehäuse aufgenommenen Ventilstift verschließbar ist, wobei dem Ventilstift eine Kolbenmanschette und eine Federeinrichtung zugeordnet ist, wobei eine Wirkfläche der Kolbenmanschette zumindest 50 Mal größer als eine Querschnittsfläche der Dosieröffnung ist. Das Ventilgehäuse kann insbesondere hülsenförmig ausgeführt sein und an einem stirnseitigen Endbereich der Hülse mit einem abschnittsweise sphärisch oder konisch geformten Wandabschnitt versehen sein, der von der Dosieröffnung durchdrungen ist. Ein Dosierraum wird von den Wandbereichen des Ventilgehäuses sowie von der Kolbenmanschette abgeschlossen und nimmt das auszutragende Medium unmittelbar vor dem Mediumaustrag auf. Die Dosieröffnung stellt somit eine kommunizierende Verbindung zwischen dem Dosierraum und der Umgebung her. Die Kolbenmanschette weist eine hydraulisch wirksame Wirkfläche auf, auf die bei Druckbeaufschlagung des in dem Dosierraum aufgenommenen Mediums eine Öffnungskraft wirkt. Die Öffnungskraft ist zumindest im Wesentlichen der durch die Federeinrichtung aufgebrachten Ventilschließkraft entgegengesetzt. Durch das extreme Verhältnis der Wirkfläche der Kolbenmanschette einerseits und der Querschnittsfläche der Dosieröffnung andererseits lässt sich auch bei geringem Druck des Mediums und hoher Schließkraft der Federeinrichtung eine zuverlässige Freigabe der Dosieröffnung durch den Ventilstift erzielen. Die Federeinrichtung ist für die Aufbringung einer hohen Ventilschließkraft ausgelegt, um zu gewährleisten, dass der Ventilstift zwischen zwei Dosiervorgängen die Dosieröffnung zuverlässig, insbesondere mikrobiologisch dicht, verschließen kann und ein Eindringen von Kontaminationen aus der Umgebung des Spenders in den Mediumspeicher nahezu ausgeschlossen werden kann. Dabei ist vorgesehen, dass zwischen dem Ventilstift und der Dosieröffnung eine Flächenpressung von zumindest 3 N/mm², bevorzugt zumindest 5 N/mm², besonders bevorzugt zumindest 6 N/mm² erreicht wird. Eine Öffnung der Ventileinrichtung ist bei einem Druck von 0,5 bar vorgesehen, die durch die Ausübung einer Betätigungskraft auf die Halteplatte durch einen Benutzer aufgebracht wird.

Bei einer bevorzugten Ausführungsform der Erfindung ist die Wirkfläche der Kolbenmanschette zumindest einhundert mal größer als die Querschnittsfläche der Dosieröffnung. Bei einer besonders bevorzugten Ausführungsform der Erfindung ist die Wirkfläche der Kolbenmanschette zumindest zweihundert mal größer als die Querschnittsfläche der Dosieröffnung. Die Federeinrichtung kann dabei insbesondere als Spiralfeder oder Wendelfeder ausgeführt sein und kann aus einem Kunststoff oder einem metallischen Werkstoff hergestellt sein.

In weiterer Ausgestaltung der Erfindung ist vorgesehen, dass der Ventilstift derart ausgeführt ist, dass er die Dosieröffnung im Schließzustand zumindest nahezu vollständig ausfüllt. Damit kann ein außenseitig in der Dosieröffnung nach Durchführung des Dosiervorgangs verbleibendes Mediumvolumen minimiert oder gänzlich vermieden werden. Kontaminationen, wie sie insbesondere durch Flüssigkeitsrückstände im Bereich der Dosieröffnung auftreten können, werden dadurch wirksam reduziert oder verhindert. Damit kann auch die Gefahr eines Eindringens von Kontaminationen in den Mediumspeicher durch reduziert werden. Derartige Kontaminationen können zwar wegen des mikrobiologisch dichten Dosierventils im Ruhezustand des Spenders verhindert werden, durch Rückströmeffekte, wie sie während des Dosiervorgangs im Bereich der Dosieröffnung auftreten können, ist jedoch eine Kontamination des Mediumspeichers denkbar. Bei einer bevorzugten Ausführungsform der Erfindung ist die Dosieröffnung als konusförmige Bohrung ausgeführt und der Dosierstift weist einen konusförmigen, stirnseitig angebrachten Fortsatz auf, der die Dosieröffnung im Schließzustand vollständig ausfüllt. Mit der konusförmigen Gestaltung der Dosieröffnung und des Dosierstifts lässt sich eine vorteilhafte Dichtwirkung erzielen. Zudem kann dadurch auch eine günstige Öffnungscharakteristik des Dosierventils erreicht werden, da bei Öffnung des Ventilspalts zwischen Dosieröffnung und Ventilstift eine durch das unter Druck stehende Medium der Schließkraft entgegenwirkende Kraft auf den konischen Fortsatz des Ventilstifts wirkt, der eine weitere Öffnung des Ventilspalts begünstigt. Bei einer bevorzugten Ausführungsform der Erfindung schließt der Ventilstift die Dosieröffnung zumindest nahezu bündig mit einer Außenoberfläche des Ventilgehäuses ab, so dass beim Schließen des Ventils überschüssiges Medium auf die Außenoberfläche des Ventilgehäuses ausgestoßen wird und dort entweder verdunsten oder abgewischt wird kann, so dass nur eine geringe oder keine Mediummenge verbleibt, die zu einer Kontamination des Ventilgehäuses im Bereich der Dosieröffnung führen würde. Bei einer besonders bevorzugten Ausführungsform der Erfindung sind für die Dosieröffnung und den Ventilstift unterschiedliche Konuswinkel vorgesehen, wobei der Konuswinkel des Ventilstifts kleiner als der Konuswinkel der Dosieröffnung gewählt wird. Dadurch lässt sich eine nahezu vollständige Ausfüllung der Dosieröffnung durch den Ventilstift mit einer geringen Dichtlänge verwirkliche. Die Dichtlänge resultiert aus dem Berührkontakt zwischen Ventilstift und Dosieröffnung, wobei insbesondere eine ringförmige Linienberührung vorliegt. Eine kurze Dichtlänge bedeutet in Anbetracht der Federkraft der Ventilfeder eine hohe Flächenpressung und gewährleistet somit eine gute mikrobiologische Abdichtung der Dosieröffnung.

Bei einer Weiterbildung der Dosierbaugruppe ist der Dosieröffnung eine Drossel vorgelagert, die einen Volumenstrom aus einem Medienspeicher zur Dosieröffnung reduziert.

Eine solche Drossel verhindert, dass nach Öffnen der Dosieröffnung ein zu schnelles Nachströmen von Medium aus dem Mediumspeicher in die Dosierbaugruppe und von dort aus durch die Dosieröffnung erfolgt. Dies verhindert einen Austrag von zuviel Medium durch eine zu hohe Kraft bei einer Betätigung. Eine Möglichkeit zur Realisierung dieser Drossel ist eine einfache Durchbrechung zwischen der Dosierbaugruppe und dem Medienspeicher, deren Durchmesser ausreichend gering gewählt ist. Der Durchmesser ist in Abhängigkeit der Viskosität des Mediums und dem gewollten Anwendungszweck festzulegen. Bei den meisten Anwendungszwecken und bei für Augentropfen üblichen Viskositäten sind Durchmesser zwischen 0,1 mm und 0,5 mm besonders zweckmäßig.

Bei einer Weiterbildung der Dosierbaugruppe ist Drossel als Kapillarrohr ausgebildet.

Bei einer solchen Ausgestaltung wird die Drossel durch ein zusätzliches Bauteil in Form eines Kapillarrohrs gebildet. Dieses wird bei der Montage in eine dafür vorgesehene Aufnahme der Dosierbaugruppe eingesetzt. Dies ist vorteilhaft, da dadurch die Fertigung der Dosierbaugruppe einfacher ist und außerdem eine besonders einfache Anpassung der Drosselwirkung durch Auswahl eines passenden Kapillarrohrs erreicht werden kann. Kapillarrohre mit Kapillaröffnungen mit Durchmessern zwischen 0,1 mm und 0,5 mm und einer Länge zwischen 2 mm und 4 mm stellen eine bevorzugte Ausführungsform dar.

An der Dosierbaugruppe kann im Bereich der Dosieröffnung eine Abtropfkante zur rückstandsfreien Ableitung von Mediumtropfen vorgesehen ist. Die Abtropfkante fördert die Bildung eines Mediumtropfens während des Dosiervorgangs und verhindert beim Abtropfen des Mediumtropfens ein Zurückbleiben größerer Mediummengen. Dadurch kann eine Kontamination im Bereich der Dosieröffnung des Spenders durch verbliebenes Medium vermieden werden. Die Abtropfkante kann insbesondere ringförmig um die Dosieröffnung angeordnet sein und mit einem spitz zulaufenden Querschnitt ausgestattet sein, um ein zumindest im Wesentlichen rückstandsfreies Abfließen des ausgetragenen Mediums sicherzustellen.

Bei einer besonders bevorzugten Ausführungsform der Erfindung ist für die Dosieröffnung ein mittlerer Durchmesser von 0,8mm, eine Länge von 1,4mm und ein Konuswinkel von 30 Grad und für den Ventilstift ein Konuswinkel von 20 Grad vorgesehen. Damit ergibt sich eine Dichtlänge von ca. 0,2mm zwischen Dosieröffnung und Ventilstift, die in Anbetracht der auf den Ventilstift wirkenden Federkräfte der Ventilfeder einen mikrobiologisch dichten Abschluss des Mediumspeichers gegenüber der Umgebung sicherstellt. Die Flächenpressung, also die auf eine Flächeneinheit wirkende Kraft zwischen den Baugruppen Dosieröffnung und Ventilstift beträgt zumindest 3 N/mm², bevorzugt zumindest 5 N/mm², besonders bevorzugt zumindest 6 N/mm². Damit kann erreicht werden, dass die Werkstoffe der Dosieröffnung und/oder des Ventilstifts im Bereich der Berührung mit der jeweils gegenüberliegenden Baugruppe nahe ihrer Fließgrenze komprimiert werden und somit auch Oberflächenrauhigkeiten derart deformiert werden, dass eine mikrobiologische Dichtigkeit gewährleistet werden kann.

Bei einer bevorzugten Ausführungsform, die für die Dosierung eines wässrigen Mediums vorgesehen ist, weist die Dosieröffnung einen konischen Querschnitt mit einem mittleren Durchmesser von ca. 1,1 mm, einer Länge von ca. 1,4mm und einem Konuswinkel von 30 Grad auf. Bei einer besonders bevorzugten Ausführungsform der Erfindung sind für die Dosieröffnung ein mittlerer Durchmesser von 0,8mm, eine Länge von 1,4mm und ein Konuswinkel von 30 Grad vorgesehen.

Weitere Vorteile und Merkmale der Erfindung ergeben sich aus den Ansprüchen sowie der nachfolgenden Beschreibung eines bevorzugten Ausführungsbeispiels der Erfindung, das anhand der Zeichnungen dargestellt ist. Dabei zeigt:
- Fig. 1: eine perspektivische Darstellung einer ersten Ausführungsform eines Spenders mit einem zwischen zwei formstabilen Halteplat- ten aufgenommenen flexiblen Mediumspeicher und einem an der einen Halteplatte vorgesehenen Dosierstutzen mit einer erfin- dungsgemäßen Dosierbaugruppe,
- Fig. 2: in einer Schnittdarstellung den Spender gemäß der Fig. 1,
- Fig. 3: in einer Schnittdarstellung den Mediumspeicher mit den Halte- platten gemäß der Fig. 2,
- Fig. 4: eine Schnittdarstellung der erfindungsgemäßen Dosierbaugrup- pe gemäß der Fig. 2,
- Fig. 5: eine Darstellung eines Dosiervorgangs am Auge eines Benut- zers mit einem Spender gemäß der Erfindung,
- Fig. 6: eine zweite Ausführungsform eines erfindungsgemäßen Spen- ders in einer Schnittdarstellung und
- Fig. 7: eine Detailansicht der in den Dosierstutzen eingesetzten Dosier- baugruppe des Spenders gemäß Fig. 6.

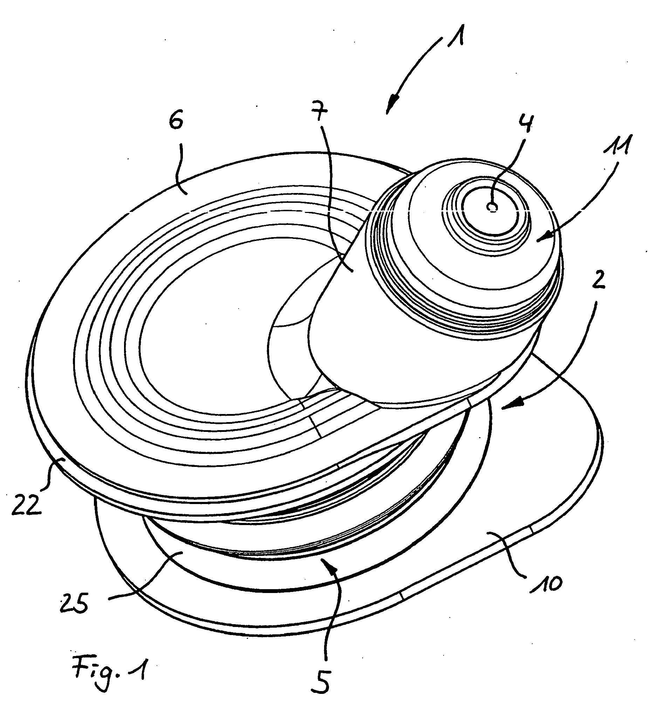

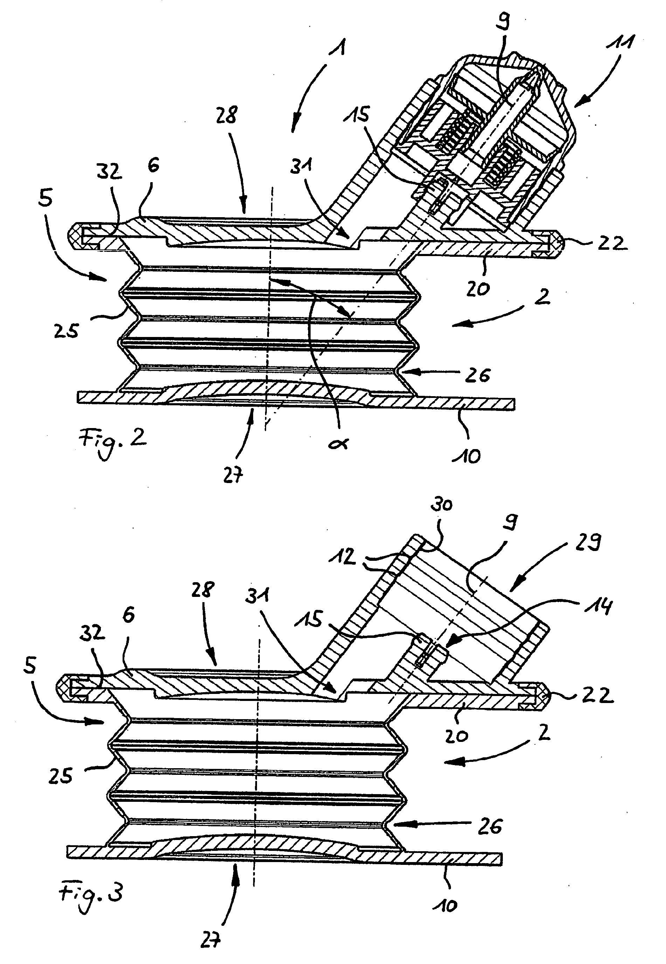

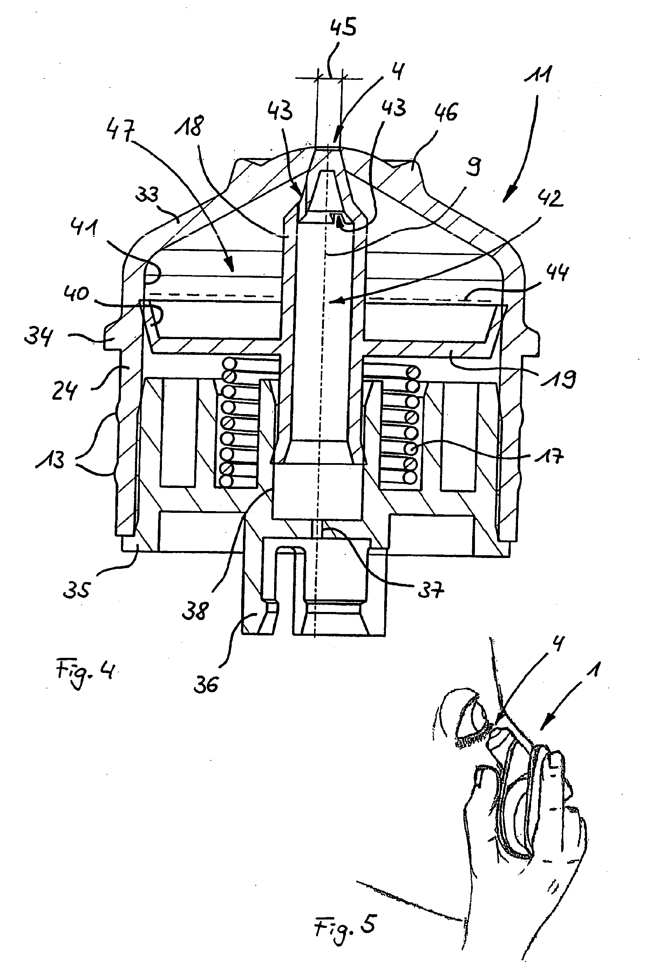

Bei dem in den Fig. 1 bis 4 dargestellten Spender 1 handelt es sich, wie in der Fig. 5 näher dargestellt, um einen Tropfer für die Dosierung von flüssigen Augentropfen. Der Spender 1 weist eine untere Halteplatte 10, eine im Wesentlichen parallel dazu angeordnete obere Halteplatte 6 sowie einen durch die Halteplatten 6, 10 und einen faltenbalgförmigen, flexibel gestalteten Wandabschnitt 5 gebildeten Mediumspeicher 2 auf. Der als Faltenbalg ausgeführte flexible Wandabschnitt 5 ist, wie in den Fig. 2 und 3 näher dargestellt, einstückig an der unteren Halteplatte 10 angeformt und weist insgesamt sechs jeweils gegenüberliegend auf einer gemeinsamen Mittelachse konzentrisch angeordnete, einstückig miteinander verbundene konusabschnittsförmige Ringbereiche 25 auf. Die Ringbereiche 25 weisen eine Wandstärke von ungefähr 0,25mm auf und sind in Verbindungszonen 26 vorzugsweise um 50% in der Wandstärke reduziert, um in der Art eines Festkörpergelenkes eine erhöhte Flexibilität zwischen den Ringbereichen 25 zu gewährleisten. Die untere Halteplatte 10 und die obere Halteplatte 6 sind jeweils mit einer Griffmulde 27, 28 versehen, die als Fingerauflage insbesondere für Daumen und Zeigefinger zur einhändigen Bedienung des Spenders 1 dienen können. An der oberen Halteplatte 6, die insbesondere aus Polypropylen hergestellt sein kann, ist ein zylindrisch geformter Dosierstutzen 7 einstückig angeformt, der in einem winkel α von 35 Grad erhaben von der Oberflache der oberen Halteplatte 6 abragt. Bei einer bevorzugten Ausführungsform der Erfindung weisen die insgesamt sechs Ringbereiche 25 des Mediumspeichers 2 einen mittleren Durchmesser von ca. 22 mm und eine Höhe von jeweils ca. 2,2mm auf, so dass sich ein Volumen des Mediumspeichers von ca. 5000mm³ oder ca. 5ml ergibt.

Wie in den Fig. 2 und 3 näher dargestellt, weist der Dosierstutzen 7 einen im Wesentlichen hohlzylindrisch geformten Aufnahmeschacht 29 auf, an dessen Innenwandung zwei zirkular umlaufende, axial voneinander beabstandete Rastnuten 12 für eine formschlüssige Aufnahme der Dosierbaugruppe 11 vorgesehen sind. Symmetrisch zu einer Mittellängsachse 9 des Dosierstutzens 7 ist ein Rastzapfen 14 angeordnet, der drei mit einer Teilung von 120 Grad koaxial zur Mittellängsachse 9 gruppierte Rastzungen 15 zur formschlüssigen Verrastung der Dosierbaugruppe 11 aufweist. Der Aufnahmeschacht 29 steht über einen Einströmkanal 31 kommunizierend mit dem Inneren des Mediumspeichers 2 in Verbindung und ermöglicht bei Aufbringung einer Betätigungskraft und einer dadurch hervorgerufenen Volumenreduktion des Mediumspeichers 2 auf die Halteplatten 6, 10 ein Abströmen des im Mediumspeicher 2 aufgenommenen Mediums in Richtung der Dosierbaugruppe 11.

Der flexibel gestaltete Wandabschnitt 5 der unteren Halteplatte 10, die insbesondere aus Polyethylen hergestellt sein kann, ist mit einem umlaufenden, formstabilen und an die Geometrie der oberen Halteplatte 6 angepassten Bund 20 versehen, der mit einer dem Mediumspeicher zugewandten Unterseite der Halteplatte 6 eine Dichtfläche 32 bildet und der in einem stirnseitigen Endbereich wie auch die Halteplatte 6 eine verjüngte, d.h. reduzierte Wandstärke aufweist. Zur sicheren Befestigung des umlaufenden Bundes 20 an der oberen Halteplatte 6 ist ein Haltering 22 stirnseitig umlaufend angespritzt, der die obere Halteplatte 6 und den umlaufenden Bund 20 bezüglich der Dichtfläche 32 kraftschlüssig und gegebenenfalls stoffschlüssig abdichtend miteinander verbindet. Zu diesem Zweck ist der Haltering 22 mit einem im Wesentlichen U-förmigen Querschnitt ausgestattet, wobei die stirnseitigen Endbereiche des umlaufenden Bundes 20 sowie der oberen Halteplatte 6 zwischen den Schenkeln des U-förmigen Querschnitts des Halterings 22 aufgenommen sind.

Die Dosierbaugruppe 11, die in der Fig. 4 näher dargestellt ist, weist ein im Wesentlichen zylinderhülsenförmig ausgeführtes Ventilgehäuse 24 auf. Das Ventilgehäuse 24 ist in einem stirnseitigen Bereich mit einem zumindest im Wesentlichen sphärisch gestalteten Wandabschnitt 33 versehen, der im Bereich der Mittellängsachse 9 von der Dosieröffnung 4 durchdrungen ist. An einer Außenseite weist das Ventilgehäuse 24 zwei zirkular umlaufende, voneinander beabstandete Rastnasen 13 auf, die für eine formschlüssige Wirkverbindung mit den Rastnuten 12 im Aufnahmeschacht 29 des Dosierstutzens 7 vorgesehen sind. Für eine Montageerleichterung und zur Sicherstellung einer korrekten Positionierung der Dosierbaugruppe 11 gegenüber dem Dosierstutzen 7 ist an dem Ventilgehäuse 24 ein zirkular umlaufender Haltebund 34 vorgesehen. Der Haltebund 34 kann mit einer Stirnseite des Dosierstutzens 7 formschlüssig in Wirkverbindung treten, um ein zu tiefes Einpressen der Dosierbaugruppe 11 in den Dosierstutzen 7 zu verhindern. In das Ventilgehäuse 24 ist ein Ventilträger 35 eingepresst, der an einem der Dosieröffnung 4 abgewandten Endbereich für eine formschlüssige Verbindung - wie in Fig. 2 näher dargestellt - mit dem Rastzapfen 14 der oberen Halteplatte 6 vorgesehene Rastzungen 36 aufweist, die symmetrisch zur Mittellängsachse 9 angeordnet sind. Eine im Ventilträger 35 angeordnete Durchgangsbohrung 37 ermöglicht einen Zustrom von Medium aus dem Mediumspeicher 2 in Richtung der Dosieröffnung 4. Die Durchgangsbohrung 37 steht mit einer Aufnahmebohrung 38 in kommunizierender Verbindung, die für eine Gleitlagerung des Ventilstifts 18 vorgesehen ist. Der Ventilstift 18 ist längs der Mittellängsachse 9 zwischen einer in Fig. 4 dargestellten Ruheposition und einer nicht dargestellten Öffnungsposition verlagerbar, um die Dosieröffnung 4 zu verschließen oder für einen Mediumaustrag freizugeben. Dem Ventilträger 35 ist eine zirkular umlaufende Aufnahmenut 39 zugeordnet, in die die als Wendelfeder ausgeführte Federeinrichtung 17 aufgenommen werden kann. Die Federeinrichtung 17 ist mit einer Vorspannung versehen und übt eine Schließkraft auf eine dem Ventilstift 18 zugeordnete Kolbenmanschette 19 in Richtung der Dosieröffnung 4 aus. Die Kolbenmanschette 19 ist rotationssymmetrisch einstückig an dem Ventilstift 18 angebracht und weist in einem Außenbereich eine zirkular umlaufende Dichtschürze 40 auf, die für eine abdichtende schiebebewegliche Wirkverbindung mit einer Zylinderwand 41 des Ventilgehäuses 24 vorgesehen ist. Der Ventilstift 18 ist mit einem Hohlraum 42 versehen, der in Austrittsöffnungen 43 mündet, die in einem der Dosieröffnung 4 zugewandten Endbereich des Ventilstifts 18 vorgesehen sind. Damit besteht eine kommunizierende Wirkverbindung zwischen dem Mediumspeicher 2 und dem vom Ventilgehäuse 24 und der Kolbenmanschette 19 gebildeten Dosierraum 47. Der Ventilstift 18 ist in einem stirnseitigen Endbereich konisch verjüngt ausgeführt, wobei ein Konuswinkel von 30 Grad vorgesehen ist und der geringste Durchmesser des Ventilstifts ca. 0,75mm beträgt.

Eine hydraulische Wirkfläche 44 der Kolbenmanschette 19 ist gegenüber einer Querschnittsfläche 45 derart ausgelegt, dass ein Flächenverhältnis von zumindest 50:1 besteht, so dass auch bei einer starken Ventilschließkraft der Federeinrichtung 17 eine Öffnung der Dosieröffnung 4 durch den Ventilstift 18 bereits bei geringem Druck erfolgen kann. Die starke Schließkraft, die von der Federeinrichtung 17 auf den Ventilstift 18 ausgeübt wird, stellt die vorteilhafte mikrobiologische Abdichtung der Dosieröffnung 4 durch den Ventilstift 18 sicher.

An einer Außenfläche des Ventilgehäuses 24 ist eine zirkular umlaufende Abtropfkante 46 vorgesehen, die einen im Wesentlichen dreieckigen Querschnitt aufweist und die gegenüber dem sphärisch gestalteten Bereich um die Dosieröffnung 4 eine scharfe Kante ausbildet, so dass bei Austrag eines Mediumtropfens aus der Dosieröffnung 4 eine vorteilhafte Tropfenbildung stattfinden kann, die zu einem zumindest im Wesentlichen rückstandsfreien Abtropfen des Mediumtropfens führt.

Der Ventilstift 18 ist in einem stirnseitigen Endbereich konisch ausgeführt und ragt nahezu vollständig in die Dosieröffnung 4 hinein, die ebenfalls eine bezogen auf die Darstellungsebene der Fig. 4 konische Kontur aufweist. Damit schließt der Ventilstift 18 nahezu bündig mit der sphärisch gestalteten Oberfläche des Wandabschnitts 33 ab, so dass bei verschlossener Dosieröffnung nur eine geringfügige Vertiefung in der Oberfläche des Wandabschnitts 33 zurückbleibt, in der sich verbleibendes Medium ansammeln kann.

Wie in der Fig. 5 dargestellt, wird zur Dosierung des Mediums in ein Auge des Benutzers mit Hilfe des Spenders 1 eine Ausrichtung der Halteplatten 6, 10 im Wesentlichen parallel zum Wangenbereich unterhalb des Auges des Benutzers gewählt. Dabei kann durch die ergonomische Gestaltung des Spenders 1 die Dosieröffnung 4 dem unteren Augenlid angenähert werden kann, so dass ein aus der Dosieröffnung 4 austretender Mediumtropfen kontrolliert in das Auge eingebracht werden kann. Die kontrollierte Einbringung wird bei dem erfindungsgemäßen Spender durch die Einhandbedienung sowie die großen Auflageflächen der Halteplatten 6, 10 und die hohe Flexibilität des faltenbalgförmig gestalteten Mediumspeichers begünstigt, hinzu tritt die durch das extreme Flächenverhältnis geprägte Ventilcharakteristik der Dosierbaugruppe 11, die bereits bei einem geringen Druck des Mediums eine Freigabe der Dosieröffnung 4 durch den Ventilstift 18 ermöglicht und somit eine besonders vorteilhafte Mediumabgabe unterstützt.

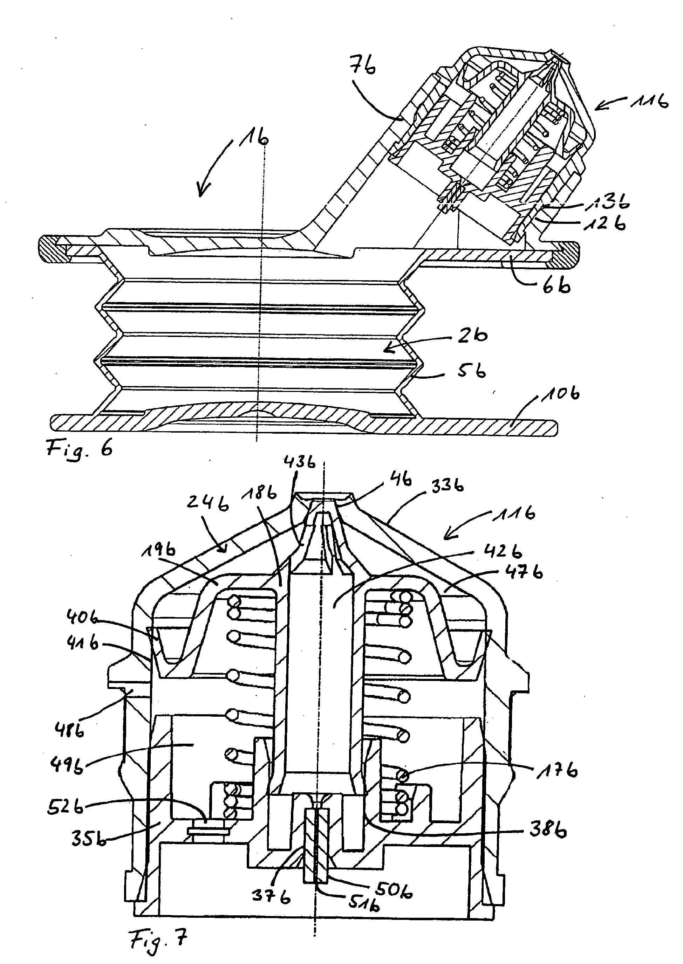

Die Fig. 6 und 7 zeigen eine zweite Ausführungsform eines erfindungsgemäßen Spenders. Die verwendeten Bezugsziffern stimmen dabei mit den Bezugsziffern der ersten Ausführungsform der Fig. 1 bis 4 überein und sind durch ein angehängten Buchstaben b gekennzeichnet.

Wie der Fig. 6 zu entnehmen ist, ist der Grundaufbau dieses Spenders 1 b im Wesentlichen der gleiche wie bei der ersten Ausführungsform.

Der Spender weist ebenfalls eine untere sowie eine dazu parallele obere Halteplatte 6b, 10b auf, zwischen denen eine Mediumspeicher 2b angeordnet ist, der durch die Halteplatten 6b, 10b sowie einen flexiblen Wandabschnitt 5b in der Art eines Faltenbalgs abgeschlossen ist. Die obere Halteplatte 6b weist einen Einströmkanal 31 b auf, der einen Durchgang vom Medienspeicher 2b zu einem zylindrischen Dosierstutzen 7b bietet. Dieser Dosierstutzen 7b erstreckt sich von der oberen Halteplatte 6b schräg nach oben. In den Dosierstutzen 7b ist eine Dosierbaugruppe 11 b eingesetzt, die im Zusammenhang mit Fig. 6 noch näher erläutert wird.

Abweichend von der Ausführungsform der Fig. 1 bis 4 ist die Dosierbaugruppe 11 b dabei ausschließlich durch Rastmittel 12b, 13b an der oberen Halteplatte 6b gehalten. Die Rastmittel 12b, 13b sind korrespondierend zueinander an einer Innenseite des Dosierstutzens 7b und an einer Außenseite der Dosierbaugruppe 11 b vorgesehen. Auf der Seite des Dosierstutzens handelt es sich um einen umlaufenden Raststeg 12b, der in eine dosierbaugruppenseitige Rastvertiefung 13b eingreift. Eine zentrische Verrastung wie durch den Rastzapfen 14 bei der Ausführungsform der Fig. 1 bis 4 ist nicht vorgesehen. Dies erleichtert die Herstellung und führt bei einer angemessenen Auslegung der außenliegenden Rastmittel 12b, 13b zu keinen Nachteilen.

Fig. 7 zeigt die Dosierbaugruppe 11 b der zweiten Ausführungsform in einer detaillierteren Ansicht. Auch die Dosierbaugruppe 11b weist nur geringfügige Unterschiede zur Dosierbaugruppe 11 der Ausführungsform der Fig. 1 bis 4 auf.

Die Dosierbaugruppe 11 b weist als wichtigste Komponenten ein Ventilgehäuse 24b, ein in das Ventilgehäuse 24b eingesetzten und relativ zum Ventilgehäuse 24b unbeweglichen Ventilträger 35b sowie einen zwischen einem distalen Stirnwandabschnitt 33b des Ventilgehäuses 24b einerseits und dem Ventilträger 35b andererseits eingesetzten Ventilstift 18b auf.

Der Ventilträger 35b weist eine zentrisch angeordnete Aufnahmebohrung 38b auf, die mit dem Ventilstift 18b zusammenwirkt. Am Grund der Aufnahmebohrung 38b ist diese mit einer Aufnahme 37b versehen, in die ein Kapillarrohr 50b eingesetzt ist. Über eine Kapillaröffnung 51 b des Kapillarrohrs 50b kann Medium aus dem Medienspeicher 2b durch den Ventilträger 35b hindurch in die Dosierbaugruppe 18b strömen. Der Ventilstift 18b ist hohlzylindrisch ausgeführt und an seinem dem Stirnwandabschnitt 33b zugewandten Ende konisch verjüngt und geschlossen ausgeführt. Vom Ventilstift 18b erstreckt sich eine Kolbenmanschette 19b radial nach außen, an deren äußeren Rand eine Dichtschürze 40b angeformt ist, die mit einer Zylinderwand 41 b der Ventilgehäuses 24b dicht abschließt. Der Ventilstift 18b wird durch eine Schraubenfeder 17b zwischen Kolbenmanschette 19b und Ventilträger 35b in Richtung des Stirnwandabschnitts 33b gedrückt. Am Stirnwandabschnitt 33b liegt der Ventilstift mit seinem konischen Ende in einer ebenfalls konisch ausgebildeten Dosieröffnung 4b an und verschließt diese mikrobiologisch dicht. Darüber hinaus schließt der Ventilstift 18b mit seinem gegenüberliegenden Ende radial umlaufend dicht mit der Aufnahmebohrung 38b des Ventilträgers 35b ab. Im Bereich seines konischen Endes weist der Ventilstift 18b eine Durchbrechung 43b auf, durch die ein schneller Druckausgleich zwischen einem Hohlraum 42b im Ventilstift 18b und einer durch die Kolbenmanschette 40b und den Stirnwandabschnitt 33b gebildeten Dosierraum 47b möglich ist.

In der Zylinderwand 41 b des Ventilgehäuses 24b ist eine Durchbrechung 48b vorgesehen, durch die eine Umgebungsdruckkammer 49b, die zwischen der Kolbenmanschette 40b und dem Ventilträger 35b angeordnet ist, druckdifferenzfrei mit der Umgebung verbunden ist. Darüber hinaus ist die Umgebungsdruckkammer 49b über eine Durchbrechung 52b mit dem Medienspeicher 2b verbunden. In diese Durchbrechung ist eine Filtermembran eingesetzt, die einen langsamen Druckausgleich zwischen der Umgebungsdruckkammer 49b sowie der Umgebung einerseits und dem Medienspeicher 2b andererseits erlaubt. Die Filtermembran dient darüber hinaus der mikrobiologischen Abdichtung des Medienspeichers 2b. Über die Umgebungsdruckkammer 49b in den Medienspeicher 2b einströmende Umgebungsluft wird gereinigt, so dass eine Kontamination des Mediums im Medienspeicher 2b vermieden wird.

Bei einer Betätigung des Spenders 1b gelangt Medium in den Hohlraum 42b sowie in den Dosierraum 47b. Gleichzeitig steigt der Druck im Medienspeicher 2b, im Hohlraum 42b sowie im Dosierraum 47b. Der Druck in der Umgebungsdruckkammer 49b bleibt derweil konstant. Ein Austreten des Mediums durch die Durchbrechung 52b findet nicht statt, da diese so geartet ist, dass die anliegende Medienflüssigkeit nicht hindurchtreten kann.

Durch die Druckdifferenz zwischen dem Dosierraum 47b und der Umgebungsdruckkammer 49b wird der Ventilstift 18b zusammen mit der Kolbenmanschette 40b in Richtung des Ventilträgers 35b verschoben. Hierdurch wird die Dosieröffnung 4b durch das ausrückende konische Ende des Ventilstifts 18b freigegeben und das im Dosierraum 47b enthaltene Medium kann austreten. Durch die Kapillaröffnung 51 b im Kapillarrohr 50b ist gewährleistet, dass auch bei sehr kräftiger Betätigung eine Begrenzung der Durchflussmenge erzielt wird. Die Kapillaröffnung 51b wirkt dabei als Drossel. Das Kapillarrohr 50b mit Kapillaröffnung 51 b dient damit dem gleichen Zweck wie die einfache Durchgangsbohrung 37 wie bei der Ausführungsform der Fig. 1 bis 4. Die Verwendung eines Kapillarrohrs hat allerdings den Vorteil, dass eine schwierige Herstellung der feinen Bohrung vermieden wird und stattdessen nur eine weniger aufwändige Aufnahme 37b für das vorgefertigte Kapillarrohr 50b geschaffen werden muss. Außerdem kann durch die Verwendung verschiedenen Kapillarrohre eine sehr einfache Anpassung des Spenders an verschiedene Medientypen und Einsatzzwecke erzielt werden.

Nach Beendigung des Austragvorgangs herrscht im Medienspeicher aufgrund des entwichenen Mediumvolumens ein Unterdruck, der mittels durch die Durchbrechung 52b und die darin eingesetzte Filtermembran einströmender Luft wieder ausgeglichen wird.

Bei einer nicht dargestellten Ausführungsform der Erfindung ist eine einstückige Gestaltung des Mediumspeichers mit beiden Halteplatten und dem an der einen Halteplatte vorgesehenen Dosierstutzen verwirklicht. Ein derartiger Mediumspeicher kann insbesondere im Blasformverfahren oder Gasinjektionsverfahren hergestellt werden, wobei gegebenenfalls eine an die einteilige Herstellung angepasste Umgestaltung des in den Fig. 1 bis 5 dargestellten Mediumspeichers vorzunehmen ist.

Bei einer weiteren nicht dargestellten Ausführungsform der Erfindung ist anstelle der Dosierbaugruppe ein elastisches Ventilelement endseitig in dem Dosierstutzen vorgesehen. Das elastische Ventilelement, das insbesondere aus einem hochelastischen Material wie Silikon hergestellt sein kann, ist von einer Wandung des Dosierstutzens eingefasst und kann insbesondere als plattenförmiges Schlitzventil ausgeführt sein. Dazu sind in einer rechteckigen, polygonen oder kreisförmigen Platte des hochelastischen Materials ein oder mehrere, insbesondere orthogonal zueinander ausgerichtete Schlitze eingeschnitten. Die Einschnitte sind so dünn ausgeführt, dass die Schnittflächen abdichtend aneinander liegen und erst bei Aufbringung eines einseitigen Überdrucks eine Auswölbung der Platte stattfindet, die zu einer Öffnung des oder der Schlitze führt. Mit Hilfe eines derartigen Ventilelements kann ein zuverlässig schließendes, einfach aufgebautes Dosierventil für den oben beschriebenen Spender verwirklicht werden. Bei einer besonders bevorzugten Ausführungsform der Erfindung ist das elastische Ventilelement in einem Ventilgehäuse aufgenommen und ist in den Dosierstutzen anstelle der Dosierbaugruppe einsetzbar.

## Patentansprüche

1. Dosierbaugruppe (11; 11 b) mit einem Ventilgehäuse (24; 24b), das in einem Wandbereich (33; 33b) von einer Dosieröffnung (4; 4b) durchdrungen ist, die von einem im Ventilgehäuse (24; 24b) aufgenommenen Ventilstift (18; 18b) verschließbar ist, wobei dem Ventilstift (18; 18b) eine Kolbenmanschette (19; 19b) und eine Federeinrichtung (17; 17b) zugeordnet ist, **dadurch gekennzeichnet, dass** eine Wirkfläche (44) der Kolbenmanschette (19; 19b) zumindest 50 mal, bevorzugt 100 mal, besonders bevorzugt 200 mal größer als eine Querschnittsfläche (45) der Dosieröffnung (4; 4b) ist.

2. Dosierbaugruppe (11; 11b) nach Anspruch 1, **dadurch gekennzeichnet, dass** der Ventilstift (18; 18b) derart ausgeführt ist, dass er die Dosieröffnung (4; 4b) im Schließzustand zumindest nahezu vollständig ausfüllt und insbesondere zumindest nahezu bündig mit einer Außenoberfläche (33; 33b) des Ventilgehäuses (24; 24b) abschließt.

3. Dosierbaugruppe (11 b) nach Anspruch 1 oder 2, **dadurch gekennzeichnet, dass** der Dosieröffnung (4; 4b) eine Drossel vorgelagert ist (37; 50b, 51 b), die einen Volumenstrom aus einem Medienspeicher (2; 2b) zur Dosieröffnung (4; 4b) reduziert.

4. Dosierbaugruppe (11 b) nach Anspruch 3,
**dadurch gekennzeichnet, dass**
die Drossel als Kapillarrohr (50b) ausgebildet ist.

## Claims

1. Dosing set (11; 11 b) having a valve housing (24; 24b) penetrated in one wall area (33; 33b) by a dosing opening (4; 4b) which is closable by a valve pin (18; 18b) held in the valve housing (24; 24b), where a piston collar (19; 19b) and a spring unit (17; 17b) are assigned to the valve pin (18; 18b), **characterized in that** a working surface (44) of the piston collar (19; 19b) is at least 50 times, preferably 100 times and particularly preferably 200 times larger than the cross-sectional area (45) of the dosing opening (4; 4b).

2. Dosing set (11; 11 b) according to Claim 1, **characterized in that** the valve pin (18; 18b) is designed such that it fills the dosing opening (4; 4b) in the closing state at least almost completely and in particular is at least in almost flush contact with an outer surface (33; 33b) of the valve housing (24; 24b).

3. Dosing set (11b) according to Claim 1 or 2, **characterized in that** a throttle (37; 50b, 51 b) is provided upstream of the dosing opening (4; 4b) and reduces a volumetric flow from a media reservoir (2; 2b) to the dosing opening (4; 4b).

4. Dosing set (11 b) according to Claim 3, **characterized in that** the throttle is designed as a capillary tube (50b).

## Revendications

1. Ensemble de dosage (11 ; 11 b) avec un corps de valve (24 ; 24b) traversé dans une zone de paroi (33 ; 33b) par un orifice de dosage (4 ; 4b) qui peut être obturé par une tige de valve (18 ; 18b) logée dans le corps de valve (24 ; 24b), sachant qu'une coupelle de piston (19 ; 19b) et un dispositif à ressort (17 ; 17b) sont associés à la tige de valve (18 ; 18b), **caractérisé en ce qu'**une surface active (44) de la coupelle de piston (19 ; 19b) est au moins 50 fois, de préférence 100 fois, en particulier de préférence 200 fois plus grande qu'une surface de la section transversale (45) de l'orifice de dosage (4 ; 4b).

2. Ensemble de dosage (11 ; 11b) selon la revendication 1, **caractérisé en ce que** la tige de valve (18 ; 18b) est conçue de manière telle, qu'à l'état fermé, elle remplit au moins presque intégralement l'orifice de dosage (4 ; 4b) et en particulier affleure au moins à peu de chose près une surface extérieure (33 ; 33b) du corps de valve (24 ; 24b).

3. Ensemble de dosage (11 b) selon la revendication 1 ou 2, **caractérisé en ce qu'**en amont de l'orifice de dosage (4 ; 4b) est placé un élément d'étranglement (37 ; 50b, 51b) qui réduit un débit volumétrique entre un réservoir de fluide (2 ; 2b) et l'orifice de dosage (4 ; 4b).

4. Ensemble de dosage (11 b) selon la revendication 3, **caractérisé en ce que** l'élément d'étranglement est formé comme tube capillaire (50b).
